# EUROPEAN PATENT SPECIFICATION

(11) **EP 1 898 876 B1**
(45) Date of publication and mention of the grant of the patent: **24.12.2008**
(21) Application number: 06754047.6
(22) Date of filing: 01.06.2006
(51) Int. Cl.: A61K 9/00, A61K 31/415, A61K 31/196, A61K 31/57

(54) **MUCOADHESIVE XYLOGLUCAN-CONTAINING FORMULATIONS USEFUL IN MEDICAL DEVICES AND IN PHARMACEUTICAL PRESENTATIONS**
MUCOADHÄSIVE XYLOGLUCAN-HALTIGE FORMULIERUNG FÜR MEDIZINPRODUKTE UND PHARMAZEUTISCHE DARREICHUNGSFORMEN
FORMULATIONS MUCOADHESIVES CONTENANT DU XYLOGLUCANE - EMPLOYÉES AUX DISPOSITIFS MEDICAUX ET AUX FORMES POSOLOGIQUES PHARMACEUTIQUES

(30) Priority: 06.06.2005 IT BO20050388
(43) Date of publication of application: 19.03.2008
(73) Proprietor: ALFA WASSERMANN S.p.A., 65020 Alanno (PE) (IT)
(72) Inventor: BOTTONI, Giuseppe, I-40133 Bologna (IT); MAFFEI, Paola, I-40133 Bologna (IT); SFORZINI, Annalisa, I-40133 Bologna (IT); FEDERICI, Mascia, I/40133 Bologna (IT); CARAMELLA, Carla, I-40133 Bologna (IT); ROSSI, Silvia, I-40133 Bologna (IT); VISCOMI, Giuseppe, Claudio, I-40133 Bologna (IT)
(74) Representative: Bianchetti, Giuseppe
(86) International application number: PCT/EP2006/005240
(87) International publication number: WO 2006/131262

(56) References cited:
- US-A1- 2004 192 640
- MIYAZAKI S ET AL: "Thermally reversible xyloglucan gels as vehicles for rectal drug delivery" JOURNAL OF CONTROLLED RELEASE, ELSEVIER SCIENCE PUBLISHERS B.V., AMSTERDAM, NL, vol. 56, no. 1-3, 4 December 1998 (1998-12-04), pages 75-83, XP004153921 ISSN: 0168-3659
- HATEFI A ET AL: "Biodegradable injectable in situ forming drug delivery systems" JOURNAL OF CONTROLLED RELEASE, ELSEVIER, AMSTERDAM, NL, vol. 80, no. 1-3, 23 April 2002 (2002-04-23), pages 9-28, XP004348621 ISSN: 0168-3659

## Description

### Field of the invention

The present invention relates to mucoadhesive formulations suitable for the application to human mucous membranes, for use as moisturizing and softening agents or as pharmaceutical release systems for local and/or systemic use.

The mucosa is a covering membrane of the internal surface of body cavities and organic channels directly or indirectly connected to the exterior, i.e. oral cavity, gastrointestinal tube, respiratory tract and vaginal cavity. Despite some differences typical of every single apparatus, mucous membranes are essentially constituted of an epithelium covering the cavity of the organ or the channel's lumen and, more deeply, of a connective tissue (*tunica propria* or mucous derma) with the supporting function; this tissue may be rich in elastic fibre and lymphocytes. Mucous membranes are wetted by the secretion of glands contained in their thickness or directly produced by the glandular epithelium which constitutes them: Such fluids mainly consist of water, glycoproteins (mucin), lipids, mineral salts and enzymes. The basic functions of all secreted fluids are to modulate interactions between epithelial cells and their environment by means of a lubrication/hydratation process, of a control process at chemical-physical and microbiological level of the "ecosystem" cavities acting also as defence barrier against pathogens.

A reduced secretion of fluids causes the inevitable onset of "dryness", a symptom connected with of a large number of pathological conditions. In fact, mucous dryness is in most cases the first clinical symptom of Sjòrgren Syndrome, a systemic autoimmune disease affecting exocrine glands causing the progressive loss of their activity. The clinical picture is characterized by reduction of lachrymal and salivar secretions (ocular dryness with keratoconjunctivitis, xerostomia, dysphagia) and reduction of vaginal mucous and bronchial tree secretions.

Vaginal mucous membrane is constituted of a non-keratinised stratified pavement epithelium. The intermediate layers of the epithelium show glycogen-rich cells. The epithelium lies on an *lamina propria* without glands and rich in vessels, most of all veins. The mucous membrane under physiological conditions is covered by a lubricating fluid mainly produced by uterine endometrium, endocervical glands (cervical mucus) and main vestibular glands (Bartolino glands) (J.R. Berman et al., Eur. Urol., 38, 20-29, 2000).

Vaginal fluid in fertile women is normally acidic (pH 3.5-4.5), while before puberty as well as after menopause secretions tend to be alkaline. The vaginal fluid is viscous, water represents the major component (90-99%), while mucin (glycoprotein) amounts to 0.5-5% by weight. Mucin is responsible for the chemical-physical characteristics of the vaginal fluid, particularly viscosity and mucoadhesive properties. Vaginal fluid also contain plasmatic proteins, enzymes (protease, betaglucoronidase, acid and alkaline phosphatase and estherase), amino acids, cholesterol, lipids, inorganic ions, lactic acid, acetic acid and desquamated epithelial cells. In addition to vaginal mucus, a serum transudate is also is present in the vagina.

Mucus production and the concentrations of the different components vary during menstrual period (M.M. Garrey, "Ginecologia Illustrata", Ed. Il Pensiero Scientifico, 1974).

The production of vaginal mucus is mainly controlled by sexual steroids which affect the biophysical properties of the secretion. Hormonal alterations can be the cause of a reduction in the mucus production with a consequent reduction of the vaginal mucous lubrication (vaginal dryness). This condition may often turn up during menopause when, as a consequence of the reduction of circulating estrogenic levels, genitals undergo anatomical modifications with a consequent change in morphological and functional characteristics. Ovaries become sclerotized, endometrium atrophises, glandular component trends to disappear, cervical channel stenotises and cervical mucus disappears, vagina narrows and loses its elasticity, vaginal walls became thinner, atrophic and dry. The regression is very evident in both vaginal and neck corions and plasmatic transudation disappears.

The reduced production of mucus exposes the mucous membrane to a greater vulnerability versus infections as a consequence of the abrasions that can occur during the sexual intercourse. Such susceptibility can be further increased by vaginal alterations (reduction of intracellular glycogen, modifications of pH and microflora) typical of menopause (G.B. Candiani, V. Danesino, A. Gastaldi, La Clinica Ostetrica e Ginecologica, Ed. Masson, 1992). This condition causes a symptomatology characterized by dyspareunia, burning and itch with not negligible psychological and sexual rebounds.

Transient, periodical or definitve vaginal dryness is a common problem that can appear at any time of life in women, from puberty to menopause and can often make sexual intercourse painful or even impossible

In addition to menopause, which evidently represents the most common condition causing vaginal dryness, many other causes exist of either endocrine (reduced levels of circulating extrogens) or non-endocrinel nature, such as hysterectomy/ovariectomy, use of oral contraceptives, amenorrhoea, vaginal mucous membrane irritation caused by microbial or chemical agents, treatments with particular drugs, Sjorgren Syndrome, diabetes mellitus and psychic alterations.

As far as oral cavity mucous membrane is concerned, fauces dryness can be the symptom of several clinical pictures related to endocrine pathologies, psychic alterations like anxiety and depression, nutritional deficiencies, cranial nerves lesions; or also the adverse side-effect of several drugs such as anti-depressants, ACE-inhibitors, chemotherapeutics/radiotherapeutics, sympathomi-metic, anti-retroviral, anxiolytic, and sedative, anti- arrhythmic, anti-inflammatory, antihypertensive, anti-histaminic, anti-ulcer and neuroleptic agents.

A reduction of the secretion also involves manifestations related to an alteration of the protective and regulative system of physiological equilibrium. For example a reduced salivary production can be responsible for oral dryness (xerostomia), taste alteration, increasing of caries onset and gingival inflammation, stomatitis and mastication problems.

Fauces dryness can also be a symptom related to clinical conditions such as subacute or chronic pharyngitis and chronic laryngitis; the etiological agents of these diseases are pathogenic microbial agents and also irritating agents like atmospheric pollutants, tobacco smoke, cold and dry air, chronic irritation caused by gastric acid material (gastroesophageal reflux), (J.Woodley, Bioadhesion, New possibilities for Drug Administrations, Clin. Pharmacokinetic, 40, 77-84, 2001).

In the light of the above considerations, it is useful to provide medical devices and pharmaceutical preparations able to reduce or to remove the negative effects directly and/or indirectly related to mucous dryness.

Moreover, since the organs, in which the mucous membranes are present, are also subjected to specific pathologies and moreover they are able to absorb drugs, it is useful to make available medicaments releasing drugs in such sites.

For such purposes bioadhesive polymers, or substances able to intimately adhere to the biological membranes, can be used, for example as disclosed in EP 0770384B1 for preparations for the vaginal use.

Xyloglucans are a class of polysaccharides structurally similar to cellulose and intimately associated to it in the cell wall in superior plants. Moreover xyloglucans are one of the main components, most likely acting as an energetic reserve, of plants seeds such as *Tamarindus Indica* original from India and South-East Asia, *Detarium senegalense* Gmelin diffused in Africa (in particular in Nigeria), *Afzelia africana* diffused in Central and East Africa and also in savannas and forests near East Africa coast and Jatoba.

Xyloglucans are characterized by a main chain of (1,4)-β-D-Glucan substituted with side chains of α-D-xylopyranose and β-D- galactopyranosyl -(1,2)-α-D-xylopyranose linked to glycan residues through a α(1,6) bond. The distribution of the residues in the side chains is different in xyloglucans of different types. Three oligomeric units have been identified in the xyloglucans structure, namely monosaccharides, octasaccharides and heptasaccharides which differ from each other in the number of galactose side chains (U. Hiroshi, Trends in Glycoscience and Glycotechnology, 14, 355-376, 2002).

The main use of xyloglucans is in human and animal nutrition. Furthermore, flour obtained by seeds of the above mentioned plants, containing xyloglucans, is commonly used in nutritional industry as a stabilizing, gelling and thickening agent.

The use of xyloglucans in medical devices or as a component in pharmaceutical preparations is also reported, although less frequently. Miyazaki et al. discloses a thermally reversible gel on the basis of xyloglucan 1-2% by weight for rectal controlled-release drug delivery (Journal of Controlled Release. 56, 1-3. 1998, 75-83). Hafeti et al refer to a biodegradable injectable xyloglucan gel, xyloglucan being present in concentrations of 0.5,1,1.5 % by weight (Journal of Controlled Release. 80, 1-3. 2002).
In US 2004/192640 an intravaginal medicament is described which contains one or more bioadhesive or mucoadhesive carriers, such as xyloglucanes or others (hydroxypropylcellulose, carbomers, alginates, pectin).
Further, for example, xyloglucans are used as viscosizing agents in products used as lachrymal substitutes, showing a safe toxicological profile even when administered to ophthalmic mucous membrane, which are particularly sensitive to lesions and irritations (M. F. Saettone et al. in US 6,056,950). WO 9729777 (M. Shozo et al.,) discloses a xyloglucan preparation, partially degraded with an enzyme such as galactosidase, for use as an ingredient in the preparation of controlled-release formulations ). According to this patent, partial removal of galactose residues from xyloglucan provides high gelling properties, so that an active principle incorporated in such gel can be gradually released in the application site.

It has now been found, and this is the object of the present invention, that preparations of suitably purified xyloglucans combined with appropriate quantities of glycerol show unexpected mucoadhesive properties. The presence of glycerol unexpectedly increases xyloglucan mucoadhesiveness.

Moreover, the glycerol content is directly related to the controlled release behaviour of this xyloglucan preparation, and mucoadhesiveness persists even reducing the viscosity of the solution.

### Description of the invention

Object of the invention are mucoadhesive and controlled release formulations consisting of aqueous solutions containing 0.05% to 5% by weight of a natural purified polymer having xyloglucan structure and 10% to 70% by weight of glycerol. These formulations are suitable for the application on human mucous membranes, such as nasal, oral and vaginal mucous membranes, as moisturizing and softening agents or as pharmaceutical release system. The combinations of the invention can be added with active principles and excipients known by those skilled in the art, for the preparation of pharmaceutical preparations or medical devices for human use that are a further object of the invention.

These pharmaceutical preparations and medical devices can be formulated as solutions, gels, ovules, sprays, mouthwashes, creams, ointments, vaginal washes and any other suitable administration systems.

A great number of active principles can be used in the present invention, preferably antibiotics, antimycotics, antiprotozoals, antivirals, anti-inflammatories, disinfectants, chemotherapeutics, analgesics, mucolytics, antitussives, decongestants, calcium absorption regulators, hormones and vaccines.

The xyloglucan purified of the present invention is obtained by purification of vegetable extracts of xyloglucans, or commercially available unfinished products with different purity grade for the use in nutritional industry.

The purification is carried out according to the following process. A 0.5% to 5% suspension, preferably 1% to 2%, of raw powder xyloglucan extract is prepared in cold distilled water. The suspension is then poured in four times the volume of boiling distilled water and the solution is kept under boiling for 15 to 30 minutes under stirring. The resulting solution is left to standleft to stand for 10 to 20 hours until the contaminant proteins and fibres precipitate. The solution is then centrifuged at 4000 - 8000 rpm for a time between 15 and 30 minutes. The supernatant is filtered through a 6 µm polypropylene filter and the filtrate is further centrifuged at 12000 - 18000 rpm for a time between 30 and 60 minutes. The supernatant can then be sterilized either by filtration through a 0.22 µm polypropylene filter or in autoclave at 121°C for a time between 20 and 40 minutes. The resulting solution can be directly used as such or lyophilized. The described process affords the purified xyloglucan in a yield between 30% and 80% in respect of the starting product.

Such purification process affords xyloglucans suitable for the pharmaceutical use, in particular providing a safety profile for use in humans, more particularly for the application to mucous membranes. The purification process of the invention provides a product with constant technological properties which guarantee reproducibility in terms of viscosity and mucoadhesiveness. Viscosity is one of more important parameters which characterize a product intended for mucoadhesive preparations. The process of the invention provides a purified xyloglucan with viscosity suitable for the scopes of invention., as determined on a 2% by weight aqueous solution by using a rotational viscosimeter at the temperature of 25°C operating at a shear rate of 200 sec⁻¹ and a rest time of 15 minutes.

The resulting viscosity values can change depending on the starting raw product. The viscosity values suitable for the use within the scopes of the present invention range between 150 and 800 mPa·sec.

Moreover, the purification process provides a remarkable reduction of protein impurities present in the starting material. Xyloglucans free from protein impurities are necessary as mucous sites are particularly sensitive to irritations. On the other hand, administration to the oral cavity can trigger anaphylactic reactions. For this reason, according to the invention an upper limit of UV absorbance at 280 nm, which is an indirect measure of residual proteins, has been introduced. The purified xyloglucans suitable for the invention should have an absorption value at 280 nm lower than 0.5 abs units when measured in a 2% by weight aqueous solution at the temperature of 25°C.

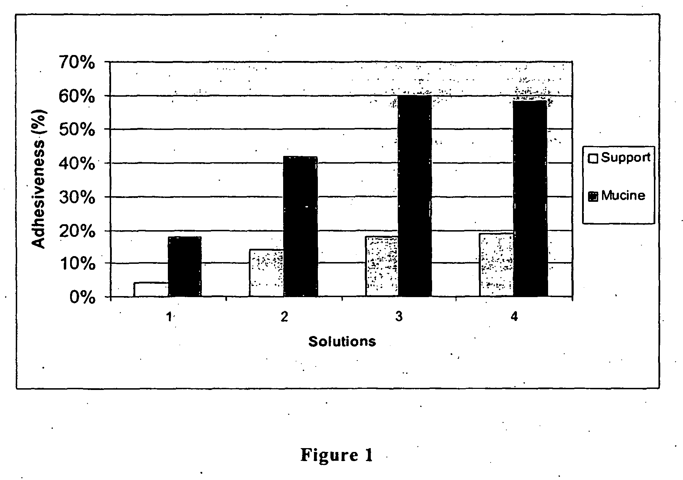

Figure 1 reports the mucoadhesiveness profile of formulations containing the purified xyloglucan at increasing glycerol concentrations, according to the data from table 2 of example 3,. Such profile shows the synergetic effect of glycerol and xyloglucan in increasing mucoadhesiveness.

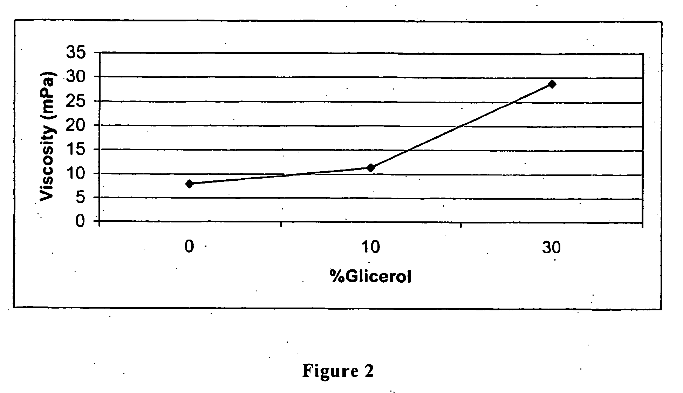

Figure 2 shows that the increase in mucoadhesiveness is not related to viscosity values of the respective solutions, according to the data from table 2 of example 3.

Finally, table 2 of example 3 shows that significant levels of mucoadhesiveness are assured also at low viscosity values, for instance at values lower than 30 mPa·sec.

The latter considerations are particularly relevant in view of the applications considered for the present invention. As a matter of fact, after administration onto mucosa, topical preparations might be subject to two phenomena both having a potentially negative impact on mucoadhesiveness: in fact near the mucous surface, the combined action of temperature and physiological fluids will induce a reduction of viscosity and a dilution effect on the preparation. Conversely, the xyloglucan-glycerol preparation of the invention, maintains mucoadhesiveness at low concentrations and at a level unrelated to viscosity.

According to the invention, the addition of glycerol in concentrations between 10% and 70% by weight to solutions of purified xyloglucan at a concentration between 0.05% and 5% by weight provides a controlled release of drugs in a manner directly related to the glycerol content and unrelated to the viscosity of the final solution.

The controlled release of drug from solutions, prepared as described in Example 2 and reported in Table 1, has been demonstrated by measuring the drug release trough Franz cell over time.

The controlled release property combined with the mucoadhesive property provides the constant, prolonged release of the active ingredient at the site of action, as reported in Table 3 for diclofenac.

The formulation containing xyloglucan and glycerol of the invention, has been evaluated for safety: the obtained results, reported in Examples 11, 12, 13, prove good local tolerability, absence of sensitisation and citotoxicity properties.

The efficacy and safety of the combination of the present invention is also shown by the clinical study reported in Example 10, wherein the use of a medical device in form of vaginal ovule afforded very good results in the treatment of dyspareunia due to vaginal dryness.

The particular mucoadhesiveness together with the controlled release properties of the products of the invention is a determinant characteristic also for the preparation of innovative pharmaceutical forms for drugs release. The advantages are connected to the possibility to keep the pharmaceutical ingredient active at the action site for longer times, increasing the drug bioavailability. Furthermore, the prolonged time of permanence, combined with the drug controlled release, allows to decrease the administration frequency. Such properties can be useful for the administration of drugs to oral, nasal and vaginal mucous membranes.

The present invention also relates to medical devices and pharmaceutical preparations containing, in addition to pharmaceutical active ingredients and excipients, 0.05% to 5% by weight of purified natural polymer having xyloglucan structure and 10% to 70% by weight of glycerol.

According to the present invention, preferred active principles are selected from antiinflammatory agents, such as diclofenac and benzidamine, hormones such as progesterone and calcitonin. Preferred formulation forms are ovules, gels, lavages and sprays.

Examples of pharmaceutical preparations according to the invention are an oropharyngeal spray containing sodium diclofenac, a vaginal gel containing progesterone, a vaginal lavage containing benzidamine hydrochloride and a nasal spray containing calcitonin.

Examples of medical devices according to the invention are a vaginal ovule having hydrating properties and an oropharyngeal spray having softening properties.

Said medical devices and pharmaceutical preparations are described in more detail in the following examples.

### Example 1

### Preparation of a purified xyloglucan

A suspension is prepared by adding 200 ml cold distilled water to 20 g Glyloid^{®} 3S (Dainippon) under powder form. The suspension is poured into 800 ml of boiling distilled water and the solution is kept under boiling for 20 minutes under magnetic stirring. The resulting solution is left to stand for 12 hours, then centrifuged at 5000 rpm for 20 minutes. The supernatant is filtered through a 6 µm polypropylene filter and the resulting solution is further centrifuged at 15000 rpm for 30 minutes. Then the solution is sterilized through a 0.22 µm polypropylene filter. Finally the resulting solution is lyophilized obtaining 14 g of purified xyloglucan preparation.

The viscosity of the purified product at the concentration of 2% by weight in an aqueous solution is 229 mPa·sec, measured at the temperature of 25 C with a "shear rate" of 200 sec⁻¹ and at a rest time of 15 minutes.

The absorbance of a 2% by weight aqueous solution at the temperature of 25°C, measured at 280 nm, is 0.2 abs units.

### Example 2

### Preparation of solutions containing sodium diclofenac

Sodium methyl-paraoxybenzoate is dissolved in water at the temperature of 70°C, then disodium monobasic phosphonate, sodium diclofenac and glycerol are added to the cooled solution. The solution is kept under stirring until complete dissolution.

Purified xyloglucan is then added and the solution is kept under stirring for 8 hours until complete swelling, then is brought to 100 ml with water.

The compositions of sodium diclofenac solutions are reported in Table 1.

**Table 1**

| Components | Solution 1 | Solution 2 | Solution 3 | Solution 4 |
|---|---|---|---|---|
| Sodium diclofenac (%w/w) | 0.08 | 0.08 | 0.08 | 0.08 |
| Purified xyloglucan (%w/w) | 0 | 0.4 | 0.4 | 0.4 |
| Glycerol (%w/w) | 30 | 0 | 10 | 30 |
| Sodium methyl-paraoxybenzoate (%w/w) | 0.15 | 0.15 | 0.15 | 0.15 |
| Disodium monobasic phosphate (%w/w) | 0.22 | 0.22 | 0.22 | 0.22 |
| Deionized water to 100 ml | | | | |

### Example 3

### Viscosity and mucoadhesiveness measure of solutions of sodium diclofenac

The viscosity of sodium diclofenac solutions prepared according to Example 2 was measured using a rheostat having a measure geometry with a cone plate C 60/1, a "shear rate" of 200 sec⁻¹ and a rest time of 3 minutes.

The *in vitro* measurement of the mucoadhesiveness of the solutions prepared according to Example 2 was carried out with the method of inclined plane as described by Sandri G. et al. (J. Pharm. Pharmacol., 56, 1083-1090, 2004). The test is carried out by gliding the sample on an inclined inert support covered with a gastric porcine mucin layer. The absorption on the mucin layer is valued referring to the absorption of the same sample on the non-covered inclined inert support.

500 mg of each sample of the solutions reported in Table 1 containing the purified xyloglucan are placed on the higher point of the plane inclined at 60° and the mixture portion gliding on the plane in a time of 2 minutes is collected and measured by weighing.

The viscosity and mucoadhesiveness values of the sodium diclofenac solutions described in Example 2 are reported in Table 2.

**Table 2**

| Solutions | Viscosity (mPa·sec) | % adhesiveness to inert support | % adhesiveness to mucin |
|---|---|---|---|
| 1 | n.d. | 4.3 | 18.0 |
| 2 | 7.86 | 14.0 | 28.0 |
| 3 | 11.32 | 17.8 | 60.0 |
| 4 | 28.83 | 18.8 | 58.2 |

### Example 4

### Evaluation of controlled release properties of a sodium diclofenac solution

The controlled release properties of a solution containing sodium diclofenac prepared according to Example 2, have been evaluated measuring the washability of the viscous solutions on Franz cell.

The solutions, in the equipment thermostated at 37°C, were washed with phosphate buffer at pH 7.4 and the quantity of active ingredient released was measured after 1, 2, 3 and 4 hours. The percentage on the total quantity of released sodium diclofenac is reported in Table 3.

**Table 3**

| Time (hours) | % sodium diclofenac Solution 2 | % sodium diclofenac Solution 3 | % sodium diclofenac Solution 4 |
|---|---|---|---|
| 1 | 31.6 | 26.7 | 6.4 |
| 2 | 46.4 | 35.5 | 14.02 |
| 3 | 48.1 | 38.0 | 24.8 |
| 4 | 52.7 | 38.0 | 28.8 |

### Example 5

### Preparation of an ovule containing purified xyloglucan having hydrating properties

*Aloe barbadensis* cryodehydrated extract, fish gelatine, sodium methyl p-hydroxybenzoate, sodium ethyl p-hydroxybenzoate and sodium propyl p-hydroxybenzoate are solubilized at 70°C in 15 ml of water. After complete solubilization, the solution is cooled to 25°C and the purified xyloglucan is added. The solution is kept under stirring for about 8 hours and after complete swelling is brought to the temperature of 45°C and glycerol is added.

After homogenisation by mixing, deionized water is added to 30 grams total weight and the product is distributed into 10 moulds.

The amounts of each component for the preparation of 10 ovules are reported in Table 4.

**Table 4**

| Component | Weight |
|---|---|
| Xyloglucan | 300 mg |
| Aloe barbadensis | 300 mg |
| Fish gelatine | 600 mg |
| Glycerol | 10.5 g |
| Sodium methyl p-hydroxybenzoate | 9.43 mg |
| Sodiumethyl p-hydroxybenzoate | 4.2 mg |
| Sodium propyl p-hydroxybenzoate | 4.81 mg |
| Deionized water to 30 grams | |

### Example 6

### Preparation of a vaginal gel containing progesterone

Methyl parahydroxybenzoate is added at 70°C in water. After complete solubilization and cooling of the solution, hydrogenated palm oil glycerides of containing the previously solubilized progesterone are added. After complete mixing, purified xyloglucan is added and the mixture it is kept under stirring for 8 hours until complete swelling of the polymer. Lastly glycerol is added and the final solution is stirred.

The amounts of the various components for the preparation of the vaginal gel are reported in Table 5.

**Table 5**

| Component | Weight |
|---|---|
| Xyloglucan | 1 g |
| Progesterone | 8 g |
| Glycerol | 30 g |
| Hydrogenated palm oil glycerides | 5 g |
| Sodium methyl para-hydroxybenzoate | 0.15 g |
| Deionized water to 56 grams | |

### Example 7

### Preparation of a vaginal lavage containing benzidamine hydrochloride

Trimethylcetylammonium p-toluenesulphonate, rose fragrance and benzidamine hydrochloride are solubilized in water. Purified xyloglucan is added after complete solubilization and the solution is kept under stirring for 8 hours until complete swelling of the polymer. Glycerol is added and the final solution is mixed until complete homogenisation.

The amounts of the various components are reported in Table 6.

**Table 6**

| Component | Weight |
|---|---|
| Xyloglucan | 1 g |
| Benzidamine hydrochloride | 100 mg |
| Glycerol | 30 g |
| Trimethylammonium p-toluenesulphonate | 5 g |
| Rose fragrance | 100 mg |
| Deionized water to 64 grams | |

### Example 8

### Preparation of a nasal spray containing calcitonin

Sodium chloride, citric acid monohydrate, sodium citrate dihydrate and hydrochloric acid are added to 65 ml of water. The mixture is kept under stirring until complete dissolution and then 10 ml of water containing the previously solubilized calcitonin are added. The purified xyloglucan is added, keeping stirring for 8 hours until complete swelling of the polymer. Then glycerol is added, the solution is mixed and water is added to the necessary weight.

The amounts of the various components are reported in Table 7.

**Table 7**

| Component | Weight |
|---|---|
| Xyloglucan | 400 mg |
| Calcitonin | 100.000 IU |
| 0.1 N Hydrochloric acid | 0.15 mg |
| Sodium chloride | 9 g |
| Sodium citrate dihydrate | 58 mg |
| Citric acid monohydrate | 58 g |
| Glycerol | 15 g |
| Deionized water to 100 grams | |

### Example 9

### Preparation of an oropharyngeal spray containing natural extracts

The natural extracts, potassium sorbate and monobasic sodium phosphate monohydrate are added to 65 ml of water. The purified xyloglucan is added, keeping stirring for 8 hours until complete dissolution. Then water is added to the necessary volume.

The amounts of the various components are reported in table 8.

**Table 8**

| Component | Weight |
|---|---|
| Xyloglucan | 400 mg |
| Erysimum dry extract | 300 mg |
| Althea dry extract | 200 mg |
| Noni dry extract | 300 mg |
| Monobasic sodium phosphate monohydrate | 220 mg |
| Potassium sorbate | 100 mg |
| Glycerol | 30 g |
| Deionized water to 100 grams | |

### Example 10

### Evaluation of the efficaciousness and tolerability of ovules containing purified xyloglucan on volunteers

The effectiveness and safety of ovules prepared according to Example 4 have been evaluated in the treatment of the dyspareunia caused by vaginal dryness in menopausal women not receving substitutive hormonal therapy.

The goal of the open study was to evaluate the treatment's efficaciousness with the ovules of Example 4 in reducing the symptom train, like dyspareunia, vulvar burning and itch, connected to the vaginal dryness.

8 Women aging between 45 and 65 years in menopause for almost 3 years and without active vaginal infections have been enrolled in the study. The treatment scheme has foreseen the administration of the ovule by vaginal route twice a day for a period of 8 days.

The clinical end point has been evaluated by using a score scale between 0 (absent symptomatology) and 5 (very intense symptomatology).

All patients have completed the treatment. The drug resulted well tolerated and efficacious both at local and systemic level, as shown in Table 9. No side reactions have been observed.

**Table 9**

| Patient (No.) | Dyspareunia | | Vulvar burning | | Vulvar itch | |
|---|---|---|---|---|---|---|
| | Basal | End of therapy | Basal | End of therapy | Basal | End of therapy |
| 1 | 4 | 1 | 5 | 3 | 3 | 1 |
| 2 | 4 | 2 | 4 | 2 | 2 | 0 |
| 3 | 5 | 2 | 4 | 1 | 2 | 0 |
| 4 | 3 | 0 | 4 | 0 | 2 | 0 |
| 5 | 4 | 1 | 4 | 1 | 1 | 1 |
| 6 | 2 | 0 | 3 | 0 | 2 | 0 |
| 7 | 3 | 0 | 2 | 0 | 3 | 1 |
| 8 | 3 | 1 | 3 | 0 | 3 | 0 |
| average | 3.50 | 0.88 | 3.63 | 0.88 | 2.25 | 0.38 |

### Example 11

### Local tolerance study of a preparation containing xyloglucan and glycerol

The local effects of the oropharyngeal spray of Example 9, on oral cavity mucous membranes were determined by examining local tissue changes after repeated placement of the test item into the cheek pouch of the Syrian hamster, over a period of 7 days.

One group constituted of 5 male and 5 female animals, was treated daily with the medical preparation at a dose of 0.5 ml for each animal for a total of 3 times in the same day with 2 hours interval, to give a total daily dosage of 1.5 ml for each animal.

Control group animals were treated with sterile water for the same period of time. No indications of a systemic effect of the test were observed during the treatment period. A slight mucous irritation was observed in animals treated with the medical preparation approximately 1 hour after the last application and no reactions were observed 24 hours later, thus proving the good tolerability in the hamster oral cavity mucous membranes.

### Example 12

### Delayed dermal sensitisation study of a preparation containing xyloglucan and glycerol

A Guinea pig model, with the test of Magnusson and Kligman (J. Invest. Dermatol., 268-276, 1969) was used to induce and elicit delayed dermal sensitisation. To induce sensitisation, a test group of 20 animals and a control group of 10 animals were intradermally injected with an emulsion of Freund's complete adjuvant, (anterior sites), the undiluted test item (middle sites) and the test item at a concentration of 50% in an emulsion of Freund's complete adjuvant (posterior sites). One week later this application was boosted by a topical application of the test item at 100% over the injection sites. Control group animals were treated with sterile water for the same period of time. Two weeks after the second induction stage, all animals were challenged by topical application of both the vehicle.

At the end of the study no response was apparent in any animal of the test or control groups and no reaction to the vehicle alone was observed in any animals.

### Example 13

### Cell toxicity assay of a solution containing xyloglucan and glycerol

The *in vitro* cytotoxicity of a solution containing xyloglucan and glycerol has been evaluated on BALB/C3T3 cells and measurement of neutral red uptake for cellular toxicity was performed on cultures of BALB/C3T3 cells treated with different concentrations of the test item and at the end of the treatment, the cultures were examined in order to evaluate changes in the morphology of the cells due to toxicity effects of the test item. Negative and positive control were included in the experiment.

The test item was solubilized in culture medium and concentrations used were 39.1; 78.1; 156; 313; 625; 1250; 2500. and 5000 micrograms/millilitre. Cytotoxicity was analysed after 24 hours treatment time. No change in the morphology of the cells and no relevant reduction in neutral red uptake was observed after treatment with the test item in any experiments at any concentration tested.

## Claims

1. Mucoadhesive formulations suitable for the application to human mucous membranes, comprising combinations of a purified natural polymer having xyloglucan structure in aqueous solution at concentrations between 0.05% and 5% by weight and glycerol at concentrations between 10% and 70% by weight.

2. Mucoadhesive formulations according to claim 1 **characterized in that** the purified natural polymer having xyloglucan structure has viscosity between 150 mPa·sec and 800 mPa·sec at the temperature of 25°C operating with a 200 sec⁻¹ shear rate and a rest time of 15 minutes and an absorbance value at 280 nm lower than 0.5 abs units in a 2% by weight aqueous solution at the temperature of 25°C.

3. Mucoadhesive formulations according to claims 1 and 2 **characterized in that** the human mucous membranes are the nasal, oropharyngeal and vaginal mucous membranes.

4. Mucoadhesive formulations according to claims 1 to 3 **characterized in that** they can contain active pharmaceutical ingredients and excipients suitable for pharmaceutical preparations and medical devices.

5. Mucoadhesive formulations according to claim 4 in form of controlled release formulations.

6. Mucoadhesive controlled release formulations according to claim 5 in which the controlled release is provided by adjusting the percentage of glycerol in the xyloglucan solution.

7. Pharmaceutical preparations and medical devices suitable for the treatment of human mucous membranes, comprising active pharmaceutical ingredients and excipients together with 0.05% to 5% by weight of a purified natural polymer having xyloglucan structure and 10% to 70% by weight of glycerol.

8. Pharmaceutical preparations and medical devices according to claim 7 **characterized in that** the purified natural polymer having xyloglucan structure has viscosity between 150 and 800 mPa_{·}sec at the temperature of 25°C operating with a 200 sec⁻¹ "shear rate" and a rest time of 15 minutes and an absorbance value at 280 nm lower than 0.5 abs in a 2% by weight aqueous solution at the temperature of 25°C.

9. Pharmaceutical preparations and medical devices according to claims 7 and 8 **characterized in that** the active principles are selected from antibiotics, antimycotics, antiprotozoals, antivirals, anti-inflammatories, disinfectants, chemotherapeutics, analgesics, antiseptics, mucolytics, antitussives, decongestants, calcium absorption and regulating agents, hormones and vaccines.

10. Pharmaceutical preparations and medical devices according to claims 7 to 9 **characterized in that** they are in the form of solutions, gels, ovules, sprays, mouthwashes, creams, ointments, unguents and lavages.

11. Pharmaceutical preparations in the form of an oropharyngeal spray containing a mucoadhesive formulation according to claims 1 and 2 together with acid diclofenac or salts thereof.

12. Pharmaceutical preparations in the form of a vaginal gel containing a mucoadhesive formulation according to claims 1 and 2 together with progesterone.

13. Pharmaceutical preparations in the form of a vaginal lavage containing a mucoadhesive formulation according to claims 1 and 2 together with benzidamine hydrochloride.

14. Pharmaceutical preparations in the form of an oral spray containing a mucoadhesive formulation according to claims 1 and 2 together with benzidamine hydrochloride.

15. Pharmaceutical preparations in the form of a nasal spray containing mucoadhesive formulation according to claims 1 and 2 together with calcitonin.

16. Medical devices in the form of a vaginal ovule having moisturizing activity containing mucoadhesive formulation according to claims 1 and 2 together with excipients.

17. Medical devices in the form of an oropharyngeal spray having softening activity containing a mucoadhesive formulation according to claims 1 and 2 together with excipients.

18. A purified natural polymer having xyloglucan structure **characterized by** viscosity values between 150 and 800 mPa_{·}sec at the temperature of 25°C by operating with a 200 sec⁻¹ shear rate and a rest time of 15 minutes and by an absorbance value at 280 nm lower than 0.5 abs in a 2% by weight aqueous solution at the temperature of 25°C.

19. A purified natural polymer having xyloglucan structure according to claim 18 **characterized in that** it is obtained from tamarind seeds.

20. A process for the purification of a natural polymer having xyloglucan structure, that consists in suspending 0.5% to 5% by weight of an extract of raw xyloglucan in a volume of cold distilled water, pouring the obtained suspension in a four time greater volume of boiling distilled water, keeping under boiling and stirring for 15 to 30 minutes, leaving to stand for a time between 10 and 20 hours, centrifuging at 4000 - 8000 rpm for a time between 15 and 30 minutes, filtering the supernatant through a 6 µm polypropylene filter, further centrifuging the filtrate at 12000 - 18000 rpm' for a time between 30 and 60 minutes and sterilizing the filtrate through a 0.22 µm polypropylene filter or in autoclave at 121°C for a time between 20 and 40 minutes.

21. A process according to clam 20 **characterized in that** the raw xyloglucan extract is an extract of tamarind seeds.

22. A process according to claim 20 **characterized in that** the raw xyloglucan extract is an extract of *Detarium senegalense Gmelin*.

23. A process according to claim 20 **characterized in that** the raw xyloglucan extract is an extract of *Afzelia africana*.

## Patentansprüche

1. Mucoadhesive Formulierungen, welche für die Anwendung an menschlichen Schleimhäuten geeignet sind, umfassend Kombinationen von einem gereinigten natürlichen Polymer mit einer Xyloglucan-Struktur in einer wässrigen Lösung bei Konzentrationen zwischen 0,05 und 5 Gew.-% und Glycerol bei Konzentrationen zwischen 10 und 70 Gew.-%.

2. Mucoadhesive Formulierungen nach Anspruch 1, **dadurch gekennzeichnet, dass** das gereinigte natürliche Polymer mit Xyloglucan-Struktur eine Viskosität zwischen 150 mPa • s und 800 mPa • s bei der Temperatur von 25 °C unter Anwendung einer Scherrate von 200 s⁻¹ und einer Restzeit von 15 Minuten und einem Absorptionswert bei 280 nm von weniger als 0,5 abs-Einheiten in einer 2 gewichtsprozentigen wässrigen Lösung bei der Temperatur von 25 °C aufweist.

3. Mucoadhesive Formulierungen nach Anspruch 1 und 2, **dadurch gekennzeichnet, dass** die menschlichen Schleimhäute nasale, oropharyngeale und vaginale Schleimhäute sind.

4. Mucoadhesive Formulierungen nach einem der Ansprüche 1 bis 3, **dadurch gekennzeichnet, dass** sie pharmazeutisch aktive Wirkstoffe und Exzipienten, welche für pharmazeutische Präparationen und medizinische Produkte geeignet sind, enthalten.

5. Mucoadhesive Formulierungen nach Anspruch 4 in der Form von Formulierungen der kontrollierten Freisetzung.

6. Mucoadhesive Formulierungen von dem Typ der kontrollierten Freisetzung nach Anspruch 5, in welchen die kontrollierte Freisetzung durch Einstellen des Gehalts an Glycerol in der Xyloglucan-Lösung erreicht wird.

7. Pharmazeutische Präparationen und medizinische Produkte, geeignet für die Behandlung von menschlichen Schleimhäuten, umfassend aktive pharmazeutische Wirkstoffe und Exzipienten zusammen mit 0,05 bis 5 Gew.-% eines gereinigten natürlichen Polymers mit Xyloglucan-Struktur und 10 bis 70 Gew.-% an Glycerol.

8. Pharmazeutische Präparationen und medizinische Produkte nach Anspruch 7, **dadurch gekennzeichnet, dass** das gereinigte natürliche Polymer mit Xyloglucan-Struktur eine Viskosität zwischen 150 und 800 mPa • s bei der Temperatur von 25 °C unter Anwendung einer Scherrate von 200 s⁻¹ und einer Restzeit von 15 Minuten und einem Absorptionswert bei 280 nm von weniger als 0,5 abs in einer 2 gewichtsprozentigen wässrigen Lösung bei der Temperatur von 25 °C aufweist.

9. Pharmazeutische Präparationen und medizinische Produkte nach Anspruch 7 oder 8, **dadurch gekennzeichnet, dass** die aktiven Wirkstoffe ausgewählt sind aus Antibiotika, Antimykotica, Antiprotozoika, antiviralen Mitteln, entzündungshemmenden Mitteln, Desinfektionsmitteln, Chemotherapeutika, Analgetika, Antiseptika, schleimlösenden Mitteln, Antitussiva, abschwellend wirkenden Mitteln, Mitteln zur Absorption und Regulierung von Calcium, Hormonen und Impfstoffen.

10. Pharmazeutische Präparationen und medizinische Vorrichtungen gemäß Anspruch 7 bis 9, **dadurch gekennzeichnet, dass** sie in der Form von Lösungen, Gelen, Ovula, Sprays, Mundwassern, Cremes, Heilsalben, Salben und Spülungen vorliegen.

11. Pharmazeutische Präparationen in der Form eines oropharyngealen Sprays, enthaltend eine mucoadhesive Formulierung gemäß den Ansprüchen 1 und 2, zusammen mit Diclofenacsäure oder Salzen davon.

12. Pharmazeutische Präparationen in der Form eines vaginalen Gels, enthaltend eine mucoadhesive Formulierung gemäß den Ansprüchen 1 und 2, zusammen mit Progesteron.

13. Pharmazeutische Präparationen in der Form einer Vaginalspülung, enthaltend eine mucoadhesive Formulierung gemäß den Ansprüchen 1 und 2, zusammen mit Benzidaminhydrochlorid.

14. Pharmazeutische Präparationen in der Form eines oralen Sprays, enthaltend eine mucoadhesive Formulierung gemäß den Ansprüchen 1 und 2, zusammen mit Benzidaminhydrochlorid.

15. Pharmazeutische Präparationen in der Form eines Nasalsprays, enthaltend eine mucoadhesive Formulierung gemäß den Ansprüchen 1 und 2, zusammen mit Calcitonin.

16. Medizinische Produkte in der Form eines Vaginalovula mit Feuchtigkeitswirkung, enthaltend eine mucoadhesive Formulierung gemäß den Ansprüchen 1 und 2, zusammen mit Exzipienten

17. Medizinische Produkte in der Form eines oropharyngealen Sprays mit weichmachender Wirkung, enthaltend eine mucoadhesive Formulierung gemäß den Ansprüchen 1 und 2, zusammen mit Exzipienten.

18. Ein gereinigtes natürliches Polymer mit Xyloglucan-Struktur, **gekennzeichnet durch** Viskositätswerte zwischen 150 und 800 mPa • s bei einer Temperatur von 25 °C unter der Einwirkung einer Scherrate von 200 s⁻¹ und einer Restzeit von 15 Minuten und bei einem Absorptionswert von 280 nm von weniger als 0,5 abs in einer 2 gewichtsprozentigen wässrigen Lösung bei einer Temperatur von 25 °C.

19. Ein gereinigtes natürliches Polymer mit Xyloglucan-Struktur gemäß Anspruch 18, **dadurch gekennzeichnet, dass** es ausgehend von Tamarind-Saatgut erhalten wird.

20. Ein Verfahren für die Reinigung eines natürlichen Polymers mit Xyloglucan-Struktur, welches besteht aus dem Suspendieren von 0,5 bis 5 Gew.-% eines Extraktes an rohem Xyloglucan in einem Volumen von kaltem destilliertem Wasser, Eingießen der erhaltenen Suspension in ein Volumen der 4-fachen Größe von siedendem destillierten Wasser, Halten unter Sieden und Rühren für 15 bis 30 Minuten, Stehenlassen für eine Zeit zwischen 10 und 20 Stunden, Zentrifugieren für eine Zeit zwischen 15 und 30 Minuten bei 4000 bis 8000 U/min, Filtrieren des Überstehenden durch einen Polypropylen-Filter von 6 µm, weiteres Zentrifugieren des Filtrats für eine Zeit zwischen 30 und 60 Minuten bei 12000 bis 18000 U/min und Sterilisieren des Filtrats durch einen Polypropylenfilter von 0,22 µm oder in einem Autoklav bei 121 °C für eine Zeit zwischen 20 und 40 Minuten.

21. Ein Verfahren gemäß Anspruch 20, **dadurch gekennzeichnet, dass** der rohe Xyloglucan-Extrakt ein Extrakt von Tamarind-Saatgut ist.

22. Ein Verfahren nach Anspruch 20, **dadurch gekennzeichnet, dass** der rohe Xyloglucan-Extrakt ein Extrakt von *Detarium senegalense Gmelin* ist.

23. Ein Verfahren nach Anspruch 20, **dadurch gekennzeichnet, dass** der rohe Xyloglucan-Extrakt ein Extrakt von *Afzelia africana* ist.

## Revendications

1. Formulations mucoadhésives appropriées pour l'application sur des membranes muqueuses humaines, comprenant des combinaisons d'un polymère naturel purifié possédant une structure de xyloglucan en solution aqueuse à des concentrations entre 0,05 % et 5 % en poids et de glycérol à des concentrations entre 10 % et 70 % en poids.

2. Formulations mucoadhésives selon la revendication 1, **caractérisées en ce que** le polymère naturel purifié possédant une structure de xyloglucan possède une viscosité entre 150 mPas et 800 mPas à la température de 25 °C, en travaillant à une vitesse de cisaillement de 200 s⁻¹ et avec un temps de repos de 15 minutes, et à une valeur d'absorbance, à 280 nm, inférieure à 0,5 unité abs. dans une solution aqueuse à 2 % en poids à la température de 25 °C.

3. Formulations mucoadhésives selon les revendications 1 et 2, **caractérisées en ce que** les membranes muqueuses humaines sont les membranes muqueuses nasale, oropharyngienne et vaginale.

4. Formulations mucoadhésives selon les revendications 1 à 3, **caractérisées en ce qu'**elles peuvent contenir des ingrédients pharmaceutiques actifs et des excipients appropriés pour des préparations pharmaceutiques et des dispositifs médicaux.

5. Formulations mucoadhésives selon la revendication 4, sous la forme de formulations à libération contrôlée.

6. Formulations mucoadhésives à libération contrôlée, selon la revendication 5, dans lesquelles la libération contrôlée est fournie en réglant le pourcentage de glycérol dans la solution de xyloglucan.

7. Préparations pharmaceutiques et dispositifs médicaux appropriés pour le traitement de membranes muqueuses humaines, comprenant des ingrédients pharmaceutiques actifs et des excipients, de manière conjointe avec un polymère naturel purifié possédant une structure de xyloglucan, à concurrence de 0,05 % à 5 % en poids, et du glycérol à concurrence de 10 % à 70 % en poids.

8. Préparations pharmaceutiques et dispositifs médicaux selon la revendication 7, **caractérises en ce que** le polymère naturel purifié possédant une structure de xyloglucan possède une viscosité entre 150 et 800 mPas à la température de 25 °C, en travaillant à une « vitesse de cisaillement » de 200 s⁻¹ et avec un temps de repos de 15 minutes, et à une valeur d'absorbance, à 280 nm, inférieure à 0,5 unité abs. dans une solution aqueuse à 2 % en poids à la température de 25 °C.

9. Préparations pharmaceutiques et dispositifs médicaux selon les revendications 7 et 8, **caractérises en ce que** les principes actifs sont choisis parmi des antibiotiques, des antimycosiques, des antiprotozoaires, des antiviraux, des anti-inflammatoires, des désinfectants, des agents chimiothérapeutiques, des analgésiques, des antiseptiques, des mucolytiques, des antitussifs, des décongestionnants, des agents d'absorption et de régulation du calcium, des hormones et des vaccins.

10. Préparations pharmaceutiques et dispositifs médicaux selon les revendications 7 à 9, **caractérises en ce qu**'ils se présentent sous la forme de solutions, de gels, de comprimés vaginaux, de sprays, de bains de bouche, de crèmes, de pommades, d'onguents et de lavages.

11. Préparations pharmaceutiques sous la forme d'un spray oropharyngien contenant une formulation mucoadhésive selon les revendications 1 et 2, de manière conjointe avec du diclofenac acide ou un de ses sels.

12. Préparations pharmaceutiques sous la forme d'un gel vaginal contenant une formulation mucoadhésive selon les revendications 1 et 2, de manière conjointe avec de la progestérone.

13. Préparations pharmaceutiques sous la forme d'un lavage vaginal contenant une formulation mucoadhésive selon les revendications 1 et 2, de manière conjointe avec du chlorhydrate de benzydamine.

14. Préparations pharmaceutiques sous la forme d'un spray oral contenant une formulation mucoadhésive selon les revendications 1 et 2, de manière conjointe avec du chlorhydrate de benzydamine.

15. Préparations pharmaceutiques sous la forme d'un spray nasal contenant une formulation mucoadhésive selon les revendications 1 et 2, de manière conjointe avec de la calcitonine.

16. Dispositifs médicaux sous la forme d'un comprimé vaginal possédant une activité humidifiante, contenant une formulation mucoadhésive selon les revendications 1 et 2, de manière conjointe avec des excipients.

17. Dispositifs médicaux sous la forme d'un spray oropharyngien possédant une activité adoucissante, contenant une formulation mucoadhésive selon les revendications 1 et 2, de manière conjointe avec des excipients.

18. Polymère naturel purifié possédant une structure de xyloglucan, **caractérisé par** des valeurs de viscosité entre 150 et 800 mPas à la température de 25 °C, en travaillant à une vitesse de cisaillement de 200 s⁻¹ et avec un temps de repos de 15 minutes et à une valeur d'absorbance, à 280 nm, inférieure à 0,5 unité abs. dans une solution aqueuse à 2 % en poids à la température de 25 °C.

19. Polymère naturel purifié possédant une structure de xyloglucan selon la revendication 18, **caractérisé en ce qu'**on l'obtient à partir de semences de tamarin.

20. Procédé pour la purification d'un polymère naturel possédant une structure de xyloglucan, qui consiste à : mettre en suspension, à concurrence de 0,5 % à 5 % en poids, un extrait de xyloglucan brut dans un volume d'eau distillée froide ; verser la suspension obtenue dans un volume quatre fois supérieur d'eau distillée en ébullition ; maintenir en ébullition et agiter pendant un laps de temps de 15 à 30 minutes ; laisser reposer pendant un laps de temps entre 10 et 20 heures ; soumettre à une centrifugation à une vitesse de 4000 à 8000 tours/minute pendant un laps de temps entre 15 et 30 minutes ; filtrer le produit surnageant à travers un filtre en polypropylène de 6 µm ; centrifuger une nouvelle fois le filtrat à une vitesse de 12.000 à 18.000 tours/minute pendant un laps de temps entre 30 et 60 minutes ; et stériliser le filtrat à travers un filtre en polypropylène de 0,22 µm ou dans un autoclave à 121 °C pendant un laps de temps entre 20 et 40 minutes.

21. Procédé selon la revendication 20, **caractérisé en ce que** l'extrait de xyloglucan brut est un extrait de semences de tamarin.

22. Procédé selon la revendication 20, **caractérisé en ce que** l'extrait de xyloglucan brut est un extrait de *Detarium senegalense Gmelin*.

23. Procédé selon la revendication 20, **caractérisé en ce que** l'extrait de xyloglucan brut est un extrait de *Afzelia africana*.
